(19) Europäisches Patentamt
European Patent Office
Office européen des brevets

(11) **EP 3 140 326 B1**

(12) **EUROPÄISCHE PATENTSCHRIFT**

(45) Veröffentlichungstag und Bekanntmachung des Hinweises auf die Patenterteilung:
**15.08.2018 Patentblatt 2018/33**

(51) Int Cl.:
***C08F 2/01*** (2006.01)      ***C08F 2/10*** (2006.01)
***C08F 220/06*** (2006.01)

(21) Anmeldenummer: **15722997.2**

(22) Anmeldetag: **07.05.2015**

(86) Internationale Anmeldenummer:
**PCT/EP2015/060090**

(87) Internationale Veröffentlichungsnummer:
**WO 2015/169913 (12.11.2015 Gazette 2015/45)**

(54) **WASSERABSORBIERENDE POLYMERPARTIKEL**

WATER ABSORBANT POLYMER PARTICLES

PARTICULES POLYMÈRES ABSORBANT L'EAU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.05.2014 EP 14167497**

(43) Veröffentlichungstag der Anmeldung:
**15.03.2017 Patentblatt 2017/11**

(73) Patentinhaber: **BASF SE**
**67056 Ludwigshafen am Rhein (DE)**

(72) Erfinder:
• **BRAUN, Markus Albert**
**68165 Mannheim (DE)**
• **HAAG, Monika**
**67061 Ludwigshafen (DE)**
• **DEUERLEIN, Stephan**
**67061 Ludwigshafen (DE)**

(56) Entgegenhaltungen:
WO-A1-2005/097313      BE-A5- 1 020 479
DE-A1-102006 037 983

**Beschreibung**

Die vorliegende Erfindung betrifft wasserabsorbierende Polymerpartikel, deren Absorptionskapazität ungeachtet einer Größen-Segregation der Polymerpartikel bei der Anwendung optimal nutzbar ist.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Polymerketten der wasserabsorbierenden Polymerpartikel sind untereinander vernetzt. Dies bewirkt unter anderem, dass die Polymerpartikel wasserunlöslich sind. Die Eigenschaften der wasserabsorbierenden Polymerpartikel können über die verwendete Vernetzermenge eingestellt werden. Mit steigender Vernetzermenge sinkt die Retentionskapazität (angegeben in Form der sogenannten Centrifuge Retention Capacity, CRC) und die Absorption unter Druck (angegeben in Form der sogenannten Absorption under Load; AUL) durchläuft ein Maximum.

Der aktuelle Trend im Windelaufbau geht dahin, dünnere Konstruktionen mit reduziertem Cellulosefaseranteil und erhöhtem Superabsorberanteil herzustellen. Der Vorteil dünnerer Konstruktionen zeigt sich nicht nur in einem verbesserten Tragekomfort, sondern auch in reduzierten Kosten bei Verpackung und Lagerhaltung. Mit dem Trend zu immer dünner werdenden Windelkonstruktionen hat sich das Anforderungsprofil an die Superabsorber deutlich verändert. Von entscheidender Bedeutung ist jetzt die Fähigkeit des Hydrogels zur Flüssigkeitsweiterleitung (Permeabilität) und -verteilung. Aufgrund der höheren Beladung des Hygieneartikels (Menge an Superabsorber pro Flächeneinheit) darf das Polymer im gequollenen Zustand keine Sperrschicht für nachfolgende Flüssigkeit bilden (Gel-Blocking). Nur wenn das Produkt gute Transporteigenschaften aufweist, kann die Retentionskapazität des gesamten Hygieneartikels optimal genutzt werden.

Neben der Permeabilität der Superabsorber (angegeben in Form der sogenannten Saline Flow Conductivity, SFC) und dem Absorptionsvermögen unter Druck ist insbesondere auch die Absorptionsgeschwindigkeit der Superabsorberpartikel (angegeben in Menge an absorbierter Flüssigkeit pro Gramm Superabsorber pro Sekunde bzw. Free Swell Rate, FSR) ein entscheidendes Kriterium, welches Aussagen darüber ermöglicht, ob ein diesen Superabsorber in großer Konzentration enthaltender absorbierender Saugkern in der Lage ist, bei seinem ersten Kontakt mit Flüssigkeiten diese schnell zu absorbieren (sogenannte "Acquisition"). Diese "Acquisition" ist bei absorbierenden Kernen mit hohem Superabsorberanteil unter anderem von der Absorptionsgeschwindigkeit des Superabsorbermaterials abhängig.

Zur Verbesserung der Anwendungseigenschaften, wie beispielsweise der Permeabilität des gequollenen Gelbetts in der Windel und der Absorption unter Druck, werden wasserabsorbierende Polymerpartikel im Allgemeinen oberflächennachvernetzt. Dadurch steigt der Vernetzungsgrad der Partikeloberfläche, wodurch die Absorption unter Druck und die Retentionskapazität zumindest teilweise entkoppelt werden können. Zur Oberflächennachvernetzung geeignete Vernetzer sind Verbindungen, die mit mindestens zwei Carboxylatgruppen der wasserabsorbierenden Polymerpartikel kovalente Bindungen bilden können.

Die Herstellung wasserabsorbierender Polymerpartikel wird beispielsweise in WO 01/038402 A1, WO 03/022896 A1, WO 03/051415 A1, WO 2006/034806 A1, WO 2006/034853 A1 DE 10 2006 037983 A, BE 1 020 479 A, WO-A-2005/097313 A, und WO 2009/115472 A1 beschrieben.

Die Extrusion der bei der Polymerisation entstehenden Polymergele wird in EP 0 497 623 A2 beschrieben. Dabei wird ein vernetztes wässriges Polymergel bei erhöhter Temperatur durch eine Lochplatte gepresst, getrocknet und gemahlen. Die so gewonnenen wasserabsorbierenden Polymerpartikel weisen einen geringen Anteil an extrahierbaren Polymeren und ein verbessertes Absorptionsverhältnis auf.

WO 2005/097313 A1 offenbart ein Verfahren zur Herstellung wasserabsorbierender Polymerpartikel, bei dem ein wässriges Polymergel verkleinert und mittels eines Extruders durch Lochplatten gepresst, getrocknet und oberflächennachvernetzt wird, wodurch Polymerpartikel mit einer hohen CRC, SFC und FSR erhalten werden.

Die ältere Anmeldung PCT/EP 2014/050980 offenbart ein Verfahren zur Herstellung wasserabsorbierender Polymerpartikel durch Polymerisation, Trocknung, Mahlung und Klassierung des erhaltenen Polymergels und Oberflächennachvernetzung. Das wässrige Polymergel wird vor der Trocknung extrudiert. Das am meisten bevorzugte Öffnungsverhältnis beträgt 10 bis 20%.

Üblicherweise weisen wasserabsorbierende Polymerpartikel keine einheitliche Partikelgröße auf, sondern eine Partikelgrößenverteilung. Bei der Anwendung, zum Beispiel in einer Windel, kommt es zu einer Segregation verschieden großer Partikel. Kleinere Teilchen können durch Lücken im Partikelgefüge oder Faserverbund schlüpfen und sich im Inneren des Hygieneartikels ansammeln. Größere Partikel sind auf der Oberseite des Artikels angereichert und kommen dementsprechend zuerst mit der zu absorbierenden Flüssigkeit in Kontakt. Quillt der Grobanteil der Partikel zu schnell an, so vermindert der gequollene Grobanteil die Flüssigkeitsweiterleitung zu den feineren Partikeln, was zu einer nur eingeschränkten Nutzung der Absorptionskapazität der Gesamtheit der Polymerpartikel führt.

Aufgabe der vorliegenden Erfindung war daher die Bereitstellung wasserabsorbierender Polymerpartikel, bei denen die kleineren Partikel eine höhere Quellgeschwindigkeit als die größeren Partikel aufweisen, bei gleichzeitig hoher Zentrifugenretentionskapazität und hoher Permeabilität des gequollenen Gelbetts.

Gelöst wurde die Aufgabe durch wasserabsorbierende Polymerpartikel, die aus vernetzten Polymerketten aufgebaut sind, die überwiegend einpolymerisierte Einheiten von zumindest teilweise neutralisierter Acrylsäure umfassen, und gekennzeichnet sind durch

a) einen Anteil von Partikeln mit einer Partikelgröße von 150 bis 300 $\mu$m von mindestens 20 Gew.-%,

b) eine Quellgeschwindigkeit der Gesamtheit der Polymerpartikel $FSR_{gesamt}$ von mindestens 0,25 g/g·s,

c) eine Differenz $\Delta FSR$ von mindestens 0,15, wobei $\Delta FSR$ definiert ist als

$$\Delta FSR = FSR_{150\text{-}300\ \mu m} - FSR_{gesamt}\ ,$$

worin $FSR_{150\text{-}300\ \mu m}$ für die Quellgeschwindigkeit der Partikel mit einer Partikelgröße von 150 bis 300 $\mu$m steht,

d) eine Zentrifugenretentionskapazität CRC von mindestens 25,0 g/g, und

e) eine Permeabilität des gequollenen Gelbetts SFC von mindestens 90 $\times$ 10$^{-7}$ CM$^3$·s/g.

Es wurde gefunden, dass eine bestimmte Differenz $\Delta FSR$ zwischen der Quellgeschwindigkeit FSR der Polymerpartikel einer Partikelgröße von 150 bis 300 $\mu$m und der FSR der Gesamtheit der Polymerpartikel für eine verbesserte Flüssigkeitsverteilung sorgt. Da der Grobanteil im Anwendungsfall langsamer quillt als das feinpartikuläre Unterbett, blockiert das Oberbett nicht oder erst später die Flüssigkeitsverteilung nach unten. Es wird somit eine bessere vertikale Flüssigkeitsverteilung in Gravitationsrichtung erreicht.

Die erfindungsgemäßen wasserabsorbierenden Polymerpartikel weisen eine Quellgeschwindigkeit $FSR_{gesamt}$ von mindestens 0,25 g/g·s, bevorzugt mindestens 0,26 g/g·s auf.

Die erfindungsgemäßen wasserabsorbierenden Polymerpartikel weisen eine Quellgeschwindigkeit der Polymerpartikel mit einer Partikelgröße von 150 bis 300 $\mu$m $FSR_{150\text{-}300\ \mu m}$ von vorzugsweise mindestens 0,35 g/g·s, bevorzugt mindestens 0,40 g/g·s auf.

Die erfindungsgemäßen wasserabsorbierenden Polymerpartikel weisen eine Differenz $\Delta FSR$ zwischen der Quellgeschwindigkeit der Polymerpartikel mit einer Partikelgröße von 150 bis 300 $\mu$m $FSR_{150\text{-}300\ \mu m}$ und der Quellgeschwindigkeit der Gesamtheit der Polymerpartikel $FSR_{gesamt}$ von mindestens 0,15, bevorzugt mindestens 0,18 auf.

Die erfindungsgemäßen wasserabsorbierenden Polymerpartikel weisen eine Zentrifugenretentionskapazität CRC von mindestens 25,0 g/g, vorzugsweise mindestens 26,0 g/g auf.

Die erfindungsgemäßen wasserabsorbierenden Polymerpartikel weisen eine Permeabilität des gequollenen Gelbetts SFC von mindestens 90 $\times$ 10$^{-7}$ cm$^3$·s/g, bevorzugt mindestens 100 $\times$ 10$^{-7}$ cm$^3$·s/g, besonders bevorzugt mindestens 105 $\times$ 10$^{-7}$ cm$^3$·s/g auf.

Erfindungsgemäße Polymerpartikel sind erhältlich durch ein Verfahren, das umfasst:

a) einen Polymerisationsschritt, bei dem eine wässrige Monomerlösung, die zumindest teilweise neutralisierte Acrylsäure, und wenigstens einen Vernetzer umfasst, polymerisiert wird, wobei man ein wässriges Polymergel erhält;

b) einen Granulationsschritt, bei dem das wässrige Polymergel mit einem Feststoffgehalt von 35 bis 70 Gew.-% und einer Temperatur von 75 bis 125 °C aus einer Zone hohen Drucks durch eine Lochplatte in eine Zone niedrigen Drucks gepresst wird und ein Granulat erhalten wird, wobei die Druckdifferenz zwischen der Zone hohen Drucks und der Zone niedrigen Drucks 4 bis weniger als 14 bar und das Öffnungsverhältnis der Lochplatte 20 bis 80% beträgt;

c) einen Trocknungsschritt, bei dem das Granulat auf einen Feuchtigkeitsgehalt von weniger als 10 Gew.-% getrocknet wird;

d) einen Mahlschritt und eine Klassierungsschritt, wobei wasserabsorbierende Polymerpartikel erhalten werden; und

e) eine Oberflächennachvernetzung der wasserabsorbierenden Polymerpartikel.

Die Eigenschaften der wasserabsorbierender Polymerpartikel lassen sich einstellen, wenn das durch Polymerisation hergestellte vernetzte Polymergel einem Granulationsschritt unterzogen wird, bei dem das Polymer unter kontrollierten Bedingungen durch eine Lochplatte gepresst wird. Die dabei auftretenden Scherkräfte führen zu einer Aufrauhung der Oberfläche der durch die Lochplatte tretenden Stränge, die zu einem Granulat zerkrümeln. Zusätzlich tritt vermutlich ein zweiter Effekt auf. Durch den Druckabfall und die hohen Temperaturen am Austritt der Lochplatte verdampft Wasser spontan aus dem wässrigen Polymergel. Es bilden sich daher Makroporen, welche zu einer Vergrößerung der Oberfläche der Polymerpartikel führen. Die veränderte Morphologie der Polymerpartikel bleibt auch nach der Trocknung, Mahlung und Klassierung erhalten und wirkt sich auf kleinere Partikel stärker aus als auf größere Partikel. Die anschließend durchgeführte Oberflächennachvernetzung führt bei derartigen kleinen Partikeln zu einem anderen Effekt als bei größeren Partikeln. Die Eindringtiefe des Oberflächennachvernetzers und die gebildete Schale erhöhter Vernetzungsdichte nahe der Oberfläche sind unabhängig von der Partikelgröße im Wesentlichen gleich groß. In Kombination mit der vergrößerten Oberfläche bewirkt dies, dass kleinere Polymerpartikel eine höhere Quellgeschwindigkeit FSR als größere Partikel aufweisen.

Die Herstellung der wasserabsorbierenden Polymerpartikel umfasst einen Polymerisationsschritt, bei dem Acrylsäure, welche zumindest teilweise neutralisiert ist, optional ein weiteres Monomer, und wenigstens ein Vernetzer in einer wässrigen Monomerlösung polymerisiert werden, wobei man ein wässriges Polymergel erhält.

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren speziell zu reinigen. Geeignete Reinigungsverfahren werden beispielsweise in der WO 02/055469 A1, der WO 03/078378 A1 und der WO 2004/035514 A1 beschrieben.

Es ist ebenfalls oft vorteilhaft, die Acrylsäure speziell zu reinigen. Geeignete Reinigungsverfahren werden beispielsweise in der WO 2004/035514 A1 beschrieben. Geeignet ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren beträgt mindestens 50 Gew.-%, bevorzugt mindestens 90 Gew.-%, besonders bevorzugt mindestens 95 Gew.-%.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 85 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen.

Die Monomere enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer. Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Die Monomerlösung kann des Weiteren ein mit der Acrylsäure/den Acrylsäuresalzen copolymerisierbares ethylenisch ungesättigtes Monomer umfassen.

Diese Monomere sind vorzugsweise wasserlöslich, d. h. die Löslichkeit in Wasser bei 23 °C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere sind beispielsweise weitere ethylenisch ungesättigte Carbonsäuren, wie Methacrylsäure und Itaconsäure, bevorzugt Methacrylsäure. Weitere geeignete Monomere sind beispielsweise ethylenisch ungesättigte Sulfonsäuren, wie Styrolsulfonsäure und 2-Acrylamido-2-methylpropansulfonsäure (AMPS).

Weitere Monomere sind beispielsweise ausgewählt unter Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Die Monomerlösung kann ein oder mehrere wasserlösliche Polymere umfassen. Als wasserlösliche Polymere können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Geeignete Vernetzer sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomers kovalente Bindungen ausbilden können. Weiterhin sind auch polyvalente Metallsalze, die mit mindestens zwei Säuregruppen des Monomers koordinative Bindungen ausbilden können, als Vernetzer geeignet.

Vernetzer sind vorzugsweise Verbindungen mit mindestens zwei polymerisierbaren Gruppen, die in das Polymernetzwerk radikalisch einpolymerisiert werden können. Geeignete Vernetzer sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 03/104299 A1, WO 03/104300 A1, WO 03/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 02/032962 A2 beschrieben.

Bevorzugte Vernetzer sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer sind die mit Acrylsäure oder Methacrylsäure zu Di- oder Triacrylaten veresterten mehrfach ethoxylierten und/oder propoxylierten Glyzerine, wie sie beispielsweise in WO 03/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer beträgt vorzugsweise 0,20 bis 1,5 Gew.-%, besonders bevorzugt 0,30 bis 1,2 Gew.-%, jeweils bezogen auf unneutralisiertes Monomer. Mit steigendem Vernetzergehalt sinkt die Zentrifugenretentionskapazität (CRC) und die Absorption unter einem Druck von 49,2 $g/cm^2$ ($AUL_{0.7psi}$) durchläuft ein Maximum.

Mit sinkender Vernetzermenge steigt der Anteil extrahierbarer Polymere (Extractables). Ein hoher Anteil extrahierbarer Polymere bedingt unter anderem eine niedrige Absorption unter einem Druck von 49,2 $g/cm^2$ $AUL_{0.7psi}$ und ein klebriges Gefühl.

Es wird eine wässrige Monomerlösung verwendet. Der Wassergehalt der Monomerlösung beträgt vorzugsweise von 40 bis 75 Gew.-%, besonders bevorzugt 45 bis 70 Gew.-%, ganz besonders bevorzugt 50 bis 65 Gew.-%. Es ist auch möglich, Monomersuspensionen, d. h. Monomerlösungen mit überschüssigem Monomer, beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Als Initiatoren können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Die bevorzugten Polymerisationsinhibitoren benötigen für eine optimale Wirkung gelösten Sauerstoff. Daher kann die Monomerlösung vor der Polymerisation durch Inertisierung, d. h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

In einer bevorzugten Ausführungsform der vorliegenden Erfindung wird die Polymerisation in Gegenwart eines inerten Gases und unter Überdruck durchgeführt.

Geeignete inerte Gase sind Stickstoff, Kohlendioxid, Wasserdampf und Argon. Die Polymerisationsreaktion wird durch Sauerstoff gehemmt. Daher sollte das inerte Gas vorzugsweise weniger als 0,001 Vol.-%, besonders bevorzugt weniger als 0,0005 Vol.-%, ganz besonders bevorzugt weniger als 0,0002 Vol.-%, Sauerstoff enthalten. Vorteilhaft wird der Polymerisationsrektor kontinuierlich von dem inerten Gas durchströmt. Der Inertgasvolumenstrom beträgt vorzugsweise von 0,001 bis 5 $m^3/h$ pro $m^3$ Reaktorvolumen, besonders bevorzugt von 0,01 bis 2 $m^3/h$ pro $m^3$ Reaktorvolumen, ganz besonders bevorzugt von 0,2 bis 1 $m^3/h$ pro $m^3$ Reaktorvolumen.

Als Inertgas wird vorzugsweise Stickstoff, besonders bevorzugt in technischer Qualität, eingesetzt. Technischer Stickstoff enthält üblicherweise mindestens 99,8 Vol.-% Stickstoff und weniger als 0,0005 Vol.-% Sauerstoff.

Der Überdruck im Polymerisationsreaktor beträgt vorzugsweise 1 bis 500 mbar, besonders bevorzugt 5 bis 100 mbar, ganz besonders bevorzugt 10 bis 30 mbar.

Die bei der Herstellung einsetzbaren Polymerisationsreaktoren weisen bevorzugt mindestens zwei achsparallel rotierende Wellen auf, wobei sich auf den Wellen üblicherweise mehrere Knet- und Transportelemente befinden.

In der Herstellung einsetzbare Polymerisationsreaktoren sind beispielsweise von der List AG (Arisdorf; Schweiz) erhältlich und in der CH 664 704 A5, EP 0 517 068 A1, WO 97/12666 A1, DE 21 23 956 A1, EP 0 603 525 A1, DE 195 36 944 A1 und DE 41 18 884 A1 beschrieben.

Solche Polymerisationsreaktoren mit mindestens zwei Wellen erzielen durch die Anordnung der Knet- und Transportelemente eine hohe Selbstreinigung, die für eine kontinuierliche Polymerisation eine wichtige Anforderung ist. Vorzugsweise rotieren die beiden Wellen gegenläufig zueinander.

Auf der Rührwelle sind die Scheibensegmente propellerartig angeordnet. Als Knet- und Transportelemente sind beispielsweise wandgängige Mischbarren sowie L- oder U-förmig ausgeformte Aufsätze geeignet.

In die wässrige Monomerlösung und/oder die polymerisierende wässrige Monomerlösung im Polymerisationsreaktor und/oder in das wässrige Polymergel können rückgeführte Polymerpartikel, deren Partikelgröße kleiner als eine vorgegebene Untergrenze sind ("Feinkorn") eingemischt werden, welche in späteren Verfahrensschritten abgetrennt wurden und/oder aus anderen Herstellungslinien stammen. Das Feinkorn kann vor oder während der Rückführung mit Wasser und/oder wässrigem Tensid angefeuchtet werden. Der Zusatz von Feinkorn erhöht den Feststoffgehalt des wässrigen Polymergels.

Wird zur Polymerisation ein Knetreaktor verwendet, so wird das Feinkorn vorzugsweise während des letzten Drittels der Polymerisation zugesetzt.

Wird das Feinkorn sehr früh zugesetzt, beispielsweise bereits zur Monomerlösung, so wird dadurch die Zentrifugenretentionskapazität CRC der erhaltenen wasserabsorbierenden Polymerpartikel gesenkt. Dies kann aber beispielsweise durch Anpassung der Einsatzmenge an Vernetzer kompensiert werden.

Das Polymergel wird in einem Granulationsschritt durch die Löcher einer Lochplatte gepresst. Die Lochöffnungen der Lochplatte sind in ihrer Form im Wesentlichen nicht beschränkt und können z. B. kreisförmig, rechteckig, dreieckig, sechseckig oder sternförmig sein. Bevorzugt sind die Lochöffnungen der Lochplatte kreisförmig. Der Durchmesser der Löcher liegt vorzugsweise im Bereich von 2 bis 20 mm, besonders bevorzugt 4 bis 15 mm, ganz besonders bevorzugt 6 bis 10 mm. Bei nicht-kreisförmigen Öffnungen ist der Lochdurchmesser als der flächenbezogene Äquivalentdurchmesser, d. h. als der Durchmesser eines Kreises gleicher Querschnittsfläche definiert.

Das Öffnungsverhältnis ist definiert als das Verhältnis der offenen Fläche (Summe der Lochflächen) der Lochplatte zur maximal nutzbaren Fläche der Lochplatte. Das Öffnungsverhältnis liegt vorzugsweise im Bereich von 20 bis 80%, bevorzugt 40 bis 70%, besonders bevorzugt 50 bis 70%.

Die Dicke der Lochplatte, d. h. die Länge der Löcher in der Lochplatte, liegt im Bereich von vorzugsweise 15 bis 45 mm, besonders bevorzugt 25 bis 40 mm. Wenn es sich bei den Löchern um Bohrungen in der Lochplatte handelt, entspricht die Dicke der Lochplatte der Länge der Löcher. Die Öffnungen können auch in Form von rohrförmigen Einsätzen in der Lochplatte verwirklicht sein, welche über die Lochplatte hinausragen können. In diesem Fall entspricht die Lochlänge der Länge der Einsätze.

Die Druckdifferenz zwischen der Zone hohen Drucks und der Zone niedrigen Drucks liegt im Bereich von vorzugsweise 4 bis weniger als 14 bar, bevorzugt 5 bis 13 bar. Eine niedrigere Druckdifferenz als die angegebene führt zu einem unerwünscht niedrigen Durchsatz des Polymergels durch die Lochplatte. Außerdem wird nur eine unzureichende Verbesserung der Quellgeschwindigkeit erzielt. Eine höhere Druckdifferenz als die angegebene führt zu einer Schädigung der inneren Struktur des Polymergels, was zu einer Erhöhung des Gehalts an extrahierbaren Polymeren und einer Beeinträchtigung der Retentionskapazität führen kann.

Bevorzugt wird der Granulationsschritt in einem Extruder durchgeführt, der ein längliches Extrudergehäuse, eine mit der Lochplatte versehene Auslassöffnung und mindestens eine im Extrudergehäuse rotierende Schneckenwelle umfasst, die das Polymergel unter Erzeugung eines Gegendrucks in Richtung der Auslassöffnung befördert. Im Allgemeinen extrudiert man das Polymergel vom hohen Druck im Inneren des Extruders durch die Lochplatte an die Umgebung, d. h. der Druck in der Zone niedrigen Drucks entspricht dem Umgebungsdruck. Um ein übermäßiges Abkühlen oder Aufheizen des Polymergels während der Extrusion zu vermeiden, wird der Extruder vorzugsweise je nach Bedarf begleitbeheizt, besonders bevorzugt mit Heizdampf, bzw. begleitgekühlt. Das Verfahren kann sowohl kontinuierlich als auch diskontinuierlich betrieben werden.

Es ist möglich, rückgeführtes Feinkorn im Extruder in das Polymergel einzuarbeiten.

Das Polymergel erfährt im Extrusionsschritt, v. a. durch die Einwirkung der rotierende(n) Schneckenwelle(n), einen mechanischen Energieeintrag. Zu hohe Energieeinträge beim Granulationsschritt führen zu einer Schädigung der inneren Struktur des Polymergels, die ihrerseits eine Verschlechterung der Quellgeschwindigkeit (FSR) und der Zentrifugenretentionskapazität (CRC) bedingt. Zu hohe Energieeinträge sind daher zu vermeiden.

Der Energieeintrag kann beispielsweise über das Verhältnis von Innenlänge zu Innendurchmesser des Extruders (L/D) beeinflusst werden. Das Verhältnis von Innenlänge zu Innendurchmesser des Extruders beträgt vorzugsweise 1 bis 6, besonders bevorzugt 2 bis 5,5, ganz besonders bevorzugt 3 bis 5.

Die bei der Extrusion eingetragene spezifische mechanische Energie (SME) beträgt vorzugsweise von 2 bis 60 kWh/t, besonders bevorzugt von 5 bis 50 kWh/t, ganz besonders bevorzugt von 10 bis 40 kWh/t. Die spezifische mechanische Energie (SME) ist die Motorleistung des Extruders in kW dividiert durch den Durchsatz an Polymergel in t/h. Dadurch wird eine Schädigung des Polymergels bei der Extrusion vermieden.

Während des Granulationsschritts b) weist das Polymergel eine Temperatur im Bereich von vorzugsweise 75 bis 125 °C, besonders bevorzugt 80 bis 120 °C, ganz besonders bevorzugt 90 bis 105 °C auf.

Der Feststoffgehalt des Polymergels beträgt vor dem Durchtritt durch die Lochplatte vorzugsweise 35 bis 70 Gew.-%, bevorzugt 40 bis 60 Gew.-%. Da, wie erläutert, der Granulationsschritt mit der Verdampfung von Wasser verbunden ist, steigt der Feststoffgehalt des Polymergels während des Granulationsschritts im Allgemeinen an. Das Verhältnis des Feststoffgehalts des Polymergels nach dem Durchtritt durch die Lochplatte zum Feststoffgehalts des Polymergels vor dem Durchtritt durch die Lochplatte ($FG_{nachExtr}/FG_{vorExtr}$) beträgt z. B. mindestens 1,01 oder mindestens 1,05 oder mindestens 1,08.

Das im Granulationsschritt erhaltene Granulat wird getrocknet. Die Trocknung des Granulats kann mittels eines beliebigen hierzu geeigneten Apparates erfolgen, vorzugsweise mittels eines Bandtrockners. Das Granulat durchläuft dabei auf einem horizontal angeordneten, perforierten Trocknungsband eine oder mehrere Trocknungskammern. Wahlweise kann zur Trocknung aber auch ein Wirbelbetttrockner oder ein Schaufeltrockner verwendet werden. Die Trocknung wird durchgeführt, bis der Restfeuchtegehalt weniger als 10 Gew.-%, zum Beispiel 1 bis weniger als 10 Gew.-%, bevorzugt 1,5 bis 8 Gew.-%, beträgt. Der Restfeuchtegehalt wird gemäß der von der EDANA empfohlenen Testmethode Nr. WSP 230.2-05 "Mass Loss Upon Heating" bestimmt. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit Partikelgrößen von weniger als 150 $\mu$m ("Feinkorn") an.

Das getrocknete Granulat wird anschließend gemahlen und klassiert. Zur Mahlung können üblicherweise ein- oder mehrstufige Walzenstühle, bevorzugt Walzenstühle, Stiftmühlen, Hammermühlen oder Schwingmühlen, besonders bevorzugt einstufige Walzenstühle eingesetzt werden. Die Klassierung umfasst mindestens zwei Siebe, wobei zusätzliche Siebe als sogenannte Entlastungssiebe eingesetzt werden können, um ein Verstopfen der Siebporen zu vermeiden. Bei der Klassierung werden sowohl Polymerpartikel, deren Partikelgröße größer als eine vorgegebene Obergrenze ist ("Grobkorn"), als auch Polymerpartikel, deren Partikelgröße kleiner als eine vorgegebene Untergrenze ist ("Feinkorn"), abgetrennt. Eine typische Obergrenze ist z. B. 710 $\mu$m; eine typische Untergrenze 150 $\mu$m.

Grobkorn senkt die Quellgeschwindigkeit. Daher sollte der Anteil an Grobkorn niedrig sein. Grobkorn wird daher üblicherweise abgetrennt und in die Mahlung des getrockneten Polymergels rückgeführt.

Feinkorn senkt die Permeabilität des gequollenen Gelbetts (SFC). Daher sollte der Anteil an Feinkorn niedrig sein.

Das Feinkorn wird daher üblicherweise abgetrennt und in das Verfahren rückgeführt, wie weiter oben ausgeführt. Es ist auch möglich in späteren Verfahrensschritten Feinkorn abzutrennen, beispielsweise nach der Oberflächennachvernetzung oder einem anderen Beschichtungsschritt. In diesem Fall ist das rückgeführte Feinkorn oberflächennachvernetzt bzw. anderweitig beschichtet, beispielsweise mit pyrogener Kieselsäure.

Die mittlere Partikelgröße der als Produktfraktion abgetrennten Polymerpartikel beträgt vorzugsweise mindestens 200 $\mu$m, besonders bevorzugt von 250 bis 600 $\mu$m, ganz besonders bevorzugt von 300 bis 500 $\mu$m. Die mittlere Partikelgröße der Produktfraktion kann mittels der von der EDANA empfohlenen Testmethode Nr. WSP 220.2-05 "Particle Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der hypothetischen, äquivalenten Maschenweite, bei der 50 Gew.-% das Sieb passieren und 50 Gew.-% auf dem Sieb lieben blieben. Praktisch kann die mittlere Partikelgröße durch eine Siebanalyse, z. B. mittels des in den nachstehenden Beispielen verwendeten Siebsatzes, ermittelt werden. Der mittlere Teilchendurchmesser ist dabei der $d_{50}$-Wert der kumulativen Partikelgrößenverteilung.

Der Anteil an Partikeln mit einer Partikelgröße von höchstens 710 $\mu$m, beträgt vorzugsweise mindestens 90 Gew.-%, besonders bevorzugt mindestens 95 Gew.-%, ganz besonders bevorzugt mindestens 98 Gew.-%.

Der Anteil an Partikeln mit einer Partikelgröße von größer 150 $\mu$m beträgt vorzugsweise mindestens 90 Gew.-%, besonders bevorzugt mindestens 95 Gew.-%, ganz besonders bevorzugt mindestens 98 Gew.-%.

Der Anteil an Partikeln mit einer Partikelgröße von 150 bis 300 $\mu$m beträgt mindestens 20 Gew.-%, vorzugsweise mindestens 25 Gew.-%, besonders bevorzugt mindestens 30 Gew.-%.

Der Anteil an Partikeln mit einer Partikelgröße von 150 bis 300 $\mu$m beträgt vorzugsweise höchstens 80 Gew.-%, bevorzugt höchstens 60 Gew.-%, besonders bevorzugt höchstens 40 Gew.-%.

Die Polymerpartikel werden zur weiteren Verbesserung der Eigenschaften oberflächennachvernetzt. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder $\beta$-Hydroxyalkylamide, wie in DE 102 04 938 A1 und US 6,239,230 beschrieben.

Des Weiteren sind in DE 40 20 780 C1 zyklische Carbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 03/031482 A1 Morpholin-2,3-dion und dessen Derivate als geeignete Oberflächennachvernetzer beschrieben.

Bevorzugte Oberflächennachvernetzer sind Ethylencarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben.

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Calcium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummonoacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,01 bis 0,8 Gew.-%, jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgebracht, z. B. aufgesprüht wird. Im Anschluss an das Aufbringen werden die mit Oberflächennachvernetzer benetzten Polymerpartikel einer thermischen Nachbehandlung unterzogen. Dabei werden die Polymerpartikel oberflächennachvernetzt und getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d. h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Reaktionstemperaturen liegen im Bereich von 100 bis 250 °C, bevorzugt 120 bis 220 °C, besonders bevorzugt 130 bis 210 °C, ganz besonders bevorzugt 150 bis 200 °C. Die bevorzugte Verweilzeit bei dieser Temperatur beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die wasserabsorbierenden Polymerpartikel nach der Oberflächennachvernetzung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die wasserabsorbierenden Polymerpartikel auf 20 bis 150 °C, vorzugsweise 30 bis 120 °C, besonders bevorzugt 40 bis 100 °C, ganz besonders bevorzugt 50 bis 80 °C, abgekühlt.

Anschließend können die oberflächennachvernetzten Polymerpartikel erneut klassiert werden, wobei Fein- bzw. Grobkorn abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80 °C, besonders bevorzugt bei 35 bis 70 °C, ganz besonders bevorzugt bei 40 bis 60 °C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 0,2 bis 10 Gew.-%, besonders bevorzugt von 0,5 bis 7,0 Gew.-%, ganz besonders bevorzugt von 1,0 bis 5,0 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Trocknung durchgeführt. Geeignete Beschichtungen zur Verbesserung der Quellgeschwindigkeit FSR sowie der Permeabilität des gequollenen Gelbettes SFC sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Ein weiterer Gegenstand der vorliegenden Erfindung sind Hygieneartikel, enthaltend erfindungsgemäße wasserabsorbierende Polymerpartikel.

Die Hygieneartikel enthalten üblicherweise eine wasserundurchlässige Rückseite, eine wasserdurchlässige Oberseite und dazwischen einen absorbierenden Kern aus den erfindungsgemäßen wasserabsorbierenden Polymerpartikeln und Fasern, vorzugsweise Cellulose. Der Anteil der erfindungsgemäßen wasserabsorbierenden Polymerpartikel im absorbierenden Kern beträgt vorzugsweise 20 bis 100 Gew.-%, bevorzugt 50 bis 100 Gew.-%.

Analysemethoden:

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (Avenue Eugene Plasky, 157, 1030 Brüssel, Belgien, www.edana.org) und INDA (1100 Crescent Green, Suite 115, Cary, North Carolina 27518, U.S.A., www.inda.org). Diese Veröffentlichung ist sowohl von EDANA als auch von INDA erhältlich.

Die Messungen sollten, wenn nicht anders angegeben, bei einer Umgebungstemperatur von 23 ± 2 °C und einer relativen Luftfeuchte von 50 ± 10% durchgeführt werden. Die wasserabsorbierenden Polymerpartikel werden vor der Messung gut durchmischt.

Partikelgrößenverteilung (Particle Size Distribution)

Die Partikelgrößenverteilung (PSD) wird gemäß der von der EDANA (European Disposables and Nonwovens Association) empfohlenen Testmethode Nr. WSP 220.2-05 "Particle Size Distribution" bestimmt. Abweichend von der Testmethode werden Siebe mit den folgenden Lochgrößen verwendet: 150, 200, 300, 500, 600 und 710 μm.

Restfeuchte

Die Restfeuchte (RF, Wassergehalt des Polymers) wird gemäß der von der EDANA (European Disposables and Nonwovens Association) empfohlenen Testmethode Nr. 230.2-05 "Moisture Content" bestimmt.

Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Absorption unter einem Druck von 49,2 g/cm$^2$ (Absorption Under Load, $AUL_{0.7psi}$)

Die Absorption unter einem Druck von 49,2 g/cm$^2$ ($AUL_{0.7psi}$) wird analog der von der EDANA empfohlenen Testmethode Nr. WSP 242.2-05 "Absorption Under Pressure, Gravimetric Determination" bestimmt, wobei statt eines Drucks von 21,1 g/cm$^2$ ($AUL_{0.3psi}$) ein Druck von 49,2 g/cm$^2$ ($AUL_{0.7psi}$) eingestellt wird.

Quellgeschwindigkeit (Free Swell Rate)

Zur Bestimmung der Quellgeschwindigkeit (FSR) werden 1,00 g (= $W_1$) der wasserabsorbierenden Polymerpartikel in ein 25 ml Becherglas eingewogen und gleichmäßig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stoppuhr gestartet. Sobald der letzte Tropfen Salzlösung absorbiert wird, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (= $W_2$). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Quellgeschwindigkeit (FSR) wie folgt:

$$\text{FSR [g/g s]} = W_2/(W_1 \times t)$$

Wenn der Feuchtegehalt der wasserabsorbierenden Polymerpartikel jedoch mehr als 3 Gew.-% beträgt, so ist das Gewicht $W_1$ um diesen Feuchtegehalt zu korrigieren:

$$W_{1,korrigiert} = W_{1, gemessen} \times (1 - RF)$$

Delta Free Swell Rate

Die Delta Free Swell Rate ($\Delta$FSR) errechnet sich nach:

$$\Delta FSR = FSR_{150\text{-}300\ \mu m} - FSR_{gesamt}$$

Flüssigkeitsweiterleitung (Saline Flow Conductivity)

Die Flüssigkeitsweiterleitung (SFC) einer gequollenen Gelschicht unter Druckbelastung von 0,3 psi (2070 Pa, 21,1 g/cm$^2$) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Flüssigkeitsweiterleitung (SFC) wird wie folgt berechnet:

$$\text{SFC [cm}^3\text{s/g]} = (Fg(t=0) \times L_0)/(d \times A \times WP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, $L_0$ die Dicke der Gelschicht in cm, d die Dichte der NaCl-Lösung in g/cm$^3$, A die Fläche der Gelschicht in cm$^2$ und WP der hydrostatische Druck über der Gelschicht in dyne/cm$^2$.

Beispiele

Vergleichsbeispiel 1

Durch kontinuierliches Mischen von Wasser, 50 gew.-%iger Natronlauge und Acrylsäure wurde eine 42,7 gew.-%ige Acrylsäure/Natriumacrylatlösung hergestellt, so dass der Neutralisationsgrad 75,0 mol-% betrug. Die Monomerlösung wurde nach dem Mischen der Komponenten durch einen Wärmetauscher kontinuierlich auf eine Temperatur von 30 °C abgekühlt und mit Stickstoff entgast. Als mehrfach ethylenisch ungesättigter Vernetzer wurde 3-fach ethoxyliertes Glyceryintriacrylat (ca. 85 gew.- %ig) verwendet. Die Einsatzmenge, bezogen auf die eingesetzte Acrylsäure (baAS), betrug 0,39 Gew.-%. Zur Initiierung der radikalischen Polymerisation wurden folgende Komponenten eingesetzt: 0,002 Gew.-% baAS Wasserstoffperoxid, zudosiert als 2,5 gew.-%ige wässrige Lösung, 0,1 Gew.-% baAS Natriumperoxodisulfat, zudosiert als 15 gew.-%ige wässrige Lösung, sowie 0,01 Gew.-% baAS Ascorbinsäure, zudosiert als 0,5 gew.-%ige wässrige Lösung. Der Durchsatz der Monomerlösung betrug 800 kg/h.

Die einzelnen Komponenten wurden kontinuierlich in einen Reaktor vom Typ List ORP 250 Contikneter (List AG, Arisdorf, Schweiz) eindosiert. In der ersten Hälfte des Reaktors wurden zusätzlich 26,3 kg/h abgetrenntes Feinkorn mit einer Partikelgröße kleiner 150 $\mu$m zugesetzt, das bei der Klassierung nach Mahlung und Trocknung des Polymers erhalten wurde.

Die Reaktionslösung hatte am Zulauf eine Temperatur von 30 °C. Die Verweilzeit der Reaktionsmischung im Reaktor betrug ca. 15 Minuten.

Das Polymergel wurde auf Trockenbleche mit Siebboden (Maschenweite 250 $\mu$m, Drahtdurchmesser 130 $\mu$m) verteilt und 70 min bei 175°C im Umlufttrockenschrank getrocknet. Die Beladung der Trockenbleche mit Polymergel betrug 0,81 g/cm$^2$.

Das getrocknete Polymergel wurde mittels eines einstufigen Walzenstuhles gemahlen (drei Mahlgänge, 1. Mahlgang mit Spaltbreite 2000 $\mu$m, 2. Mahlgang mit Spaltbreite 600 $\mu$m und 3. Mahlgang mit Spaltbreite 400 $\mu$m). Das gemahlene getrocknete Polymergel wurde klassiert und eine synthetische Partikelgrößenverteilung (PSD) mit folgender Zusammensetzung abgemischt:

> 500 bis 710 $\mu$m: 15,7 Gew.-%
> 300 bis 500 $\mu$m: 56,6 Gew.-%
> 150 bis 300 $\mu$m: 27,7 Gew.-%

Ein Teil des Feinkorns (Siebfraktion mit einer Partikelgröße < 150 $\mu$m) wurde verwendet, um es bei der Polymerisation in den Reaktor mit einzudosieren.

1200 g des getrockneten Polymers wurden zur Oberflächennachvernetzung in einem Pflugschar®-Mischer mit Heizmantel vom Typ M5 (Gebr. Lödige Maschinenbau GmbH, Paderborn, Deutschland) bei 23 °C und einer Wellendrehzahl von 200 Umdrehungen pro Minute mittels einer Zweistoff-Sprühdüse mit folgender Lösung beschichtet (jeweils bezogen auf das Grundpolymer):

> 0,994 Gew.-%    Isopropanol
> 0,14 Gew.-%    einer Lösung aus 50 Gew.-% 1,3-Propandiol und 50 Gew.-% N-(2-Hydroxyethyl)-2-oxazolidinon
> 0,431 Gew.-%    entsalztes Wasser
> 0,70 Gew.-%    1,2-Propandiol
> 2,273 Gew.-%    einer 22 gew.-%igen wässrigen Aluminiumlaktatlösung
> 0,004 Gew.-%    Sorbitan-Monolaurat (Span 20, Croda international PLC)

Nach dem Aufsprühen wurde das Produkt aus dem Mischer entnommen und in einen zweiten Mischer desselben Typs, der mit 238 °C heißer Heizflüssigkeit vorgeheizt worden war, überführt. Die Wellendrehzahl wurde auf 50 Umdrehungen pro Minute eingestellt und das Produkt auf eine Produkttemperatur von 185 °C gebracht. Um diese Temperatur zu halten, wurde die Temperatur der Heizflüssigkeit passend reduziert. Dem Reaktionsgemisch wurden beginnend mit der 25. Minute nach Polymereinfüllen alle 5 Minuten Proben von jeweils etwa 20 g entnommen. Insgesamt wurden 10 Proben entnommen. Die Produktproben wurden über ein Sieb mit einer Maschenweite von 710 $\mu$m abgesiebt, um Agglomerate zu entfernen. Es wurden die SFC-Werte aller Proben bestimmt. Der Vergleichbarkeit halber wurden die Proben ausgewählt, deren SFC-Wert am nächsten bei 100 (cm$^3$·s)/10$^7$ g lag. Ihre Eigenschaften sind in Tabelle 1 angegeben.

Vergleichsbeispiel 2

Eine Probe des kommerziell erhältlichen Superabsorber ASAP 535 (BASF SE, 67056 Ludwigshafen, Deutschland) hatte die folgende Partikelgrößenverteilung:

500 bis 710 $\mu$m: 35,4 Gew.-%
300 bis 500 $\mu$m: 56,3 Gew.-%
150 bis 300 $\mu$m: 8,3 Gew.-%

Dieser Superabsorber hat die in Tabelle 1 zusammengefassten Eigenschaften.

Vergleichsbeispiel 3

Der in Vergleichsbeispiel 2 beschriebene Superabsorber wurde gesiebt, fraktioniert und folgende künstliche Partikelgrößenverteilung eingestellt:

500 bis 710 $\mu$m: 40,5 Gew.-%
300 bis 500 $\mu$m: 32,3 Gew.-%
150 bis 300 $\mu$m: 27,2 Gew.-%

Dieser Superabsorber hat die in Tabelle 1 zusammengefassten Eigenschaften.

Beispiel 1

Durch kontinuierliches Mischen von Wasser, 50 gew.-%iger Natronlauge und Acrylsäure wurde eine 42,7 gew.-%ige Acrylsäure/Natriumacrylatlösung hergestellt, so dass der Neutralisationsgrad 75,0 mol-% betrug. Die Monomerlösung wurde nach dem Mischen der Komponenten durch einen Wärmetauscher kontinuierlich auf eine Temperatur von 30 °C abgekühlt und mit Stickstoff entgast. Als mehrfach ethylenisch ungesättigter Vernetzer wurde 3-fach ethoxyliertes Glyceryintriacrylat (ca. 85 Gew.-%ig) verwendet. Die Einsatzmenge, bezogen auf die eingesetzte Acrylsäure (baAS), betrug 0,39 Gew.-%. Zur Initiierung der radikalischen Polymerisation wurden folgende Komponenten eingesetzt: 0,002 Gew.-% baAS Wasserstoffperoxid, zudosiert als 2,5 gew.-%ige wässrige Lösung, 0,1 Gew.-% baAS Natriumperoxodisulfat, zudosiert als 15 gew.-%ige wässrige Lösung, sowie 0,01 Gew.-% baAS Ascorbinsäure, zudosiert als 0,5 gew.-%ige wässrige Lösung. Der Durchsatz der Monomerlösung betrug 800 kg/h.
Die einzelnen Komponenten wurden kontinuierlich in einen Reaktor vom Typ List ORP 250 Contikneter (List AG, Arisdorf, Schweiz) eindosiert. In der ersten Hälfte des Reaktors wurden zusätzlich 26,3 kg/h abgetrenntes Feinkorn mit einer Partikelgröße kleiner 150 $\mu$m zugesetzt, das bei der Klassierung nach Mahlung und Trocknung des Polymers erhalten wurde.
Die Reaktionslösung hatte am Zulauf eine Temperatur von 30 °C. Die Verweilzeit der Reaktionsmischung im Reaktor betrug ca. 15 Minuten.
Das so erhaltene Polymergel wurde mit einem Extruder vom Typ ECT EXR T80 (ECT GmbH, Mühlacker, Deutschland) extrudiert. Die Temperatur des Polymergels bei der Extrusion betrug 86 °C. Der Extrudermantel wurde mit 150 °C heißem Öl beheizt. Die Lochplatte mit einem Durchmesser von 80 mm hatte 66 Löcher mit einem Lochdurchmesser von je 8 mm. Die Dicke der Lochplatte betrug 33 mm. Das Verhältnis von Innenlänge zu Innendurchmesser des Extruders (L/D) betrug 4. Die Rotationsgeschwindigkeit der Extruderschnecke betrug 100 Umdrehungen pro Minute.
Das Polymergel wurde auf Trockenbleche mit Siebboden (Maschenweite 250 $\mu$m, Drahtdurchmesser 130 $\mu$m) verteilt und 70 min bei 175 °C im Umlufttrockenschrank getrocknet. Die Beladung der Trockenbleche mit Polymergel betrug 0,81 g/cm$^2$.
Das getrocknete Polymergel wurde mittels eines einstufigen Walzenstuhles gemahlen (drei Mahlgänge, 1. Mahlgang mit Spaltbreite 2000 $\mu$m, 2. Mahlgang mit Spaltbreite 600 $\mu$m und 3. Mahlgang mit Spaltbreite 400 $\mu$m). Das gemahlene getrocknete Polymergel wurde klassiert und eine synthetische Partikelgrößenverteilung (PSD) mit folgender Zusammensetzung abgemischt:

500 bis 710 $\mu$m: 18,6 Gew.-%
300 bis 500 $\mu$m: 55,8 Gew.-%
150 bis 300 $\mu$m: 25,6 Gew.-%

Ein Teil des Feinkorns (Siebfraktion mit einer Partikelgröße <150 $\mu$m) wurde verwendet, um es bei der Polymerisation in den Reaktor mit einzudosieren.

1200 g des getrockneten Polymers wurden zur Oberflächennachvernetzung in einem Pflugschar®-Mischer mit Heizmantel vom Typ M5 (Gebr. Lödige Maschinenbau GmbH, Paderborn, Deutschland) bei 23 °C und einer Wellendrehzahl von 200 Umdrehungen pro Minute mittels einer Zweistoff-Sprühdüse mit folgender Lösung beschichtet (jeweils bezogen auf das Grundpolymer):

| | |
|---|---|
| 0,994 Gew.-% | Isopropanol |
| 0,14 Gew.-% | einer Lösung aus 50 Gew.-% 1,3-Propandiol und 50 Gew.-% N-(2-Hydroxyethyl)-2-oxazolidinon |
| 0,431 Gew.-% | entsalztes Wasser |
| 0,70 Gew.-% | 1,2-Propandiol |
| 2,273 Gew.-% | einer 22 gew.-%igen wässrigen Aluminiumlaktatlösung |
| 0,004 Gew.-% | Sorbitan-Monolaurat (Span 20, Croda International PLC) |

Nach dem Aufsprühen wurde das Produkt aus dem Mischer entnommen und in einen zweiten Mischer desselben Typs, der mit 238 °C heißer Heizflüssigkeit vorgeheizt worden war, überführt. Die Wellendrehzahl wurde auf 50 Umdrehungen pro Minute eingestellt und das Produkt auf eine Produkttemperatur von 185 °C gebracht. Um diese Temperatur zu halten, wurde die Temperatur der Heizflüssigkeit passend reduziert. Dem Reaktionsgemisch wurden beginnend mit der 25. Minute nach Polymereinfüllen alle 5 Minuten Proben von jeweils etwa 20 g entnommen. Insgesamt wurden 10 Proben entnommen. Die Produktproben wurden über ein Sieb mit einer Maschenweite von 710 $\mu$m abgesiebt, um Agglomerate zu entfernen. Es wurden die SFC-Werte aller Proben bestimmt. Der Vergleichbarkeit halber wurden die Proben ausgewählt, deren SFC-Wert am nächsten bei 100 (cm³·s)/10⁷ g lag. Ihre Eigenschaften sind in Tabelle 1 angegeben.

Beispiel 2

Durch kontinuierliches Mischen von Wasser, 50 gew.-%iger Natronlauge und Acrylsäure wurde eine 42,7 gew.-%ige Acrylsäure/Natriumacrylatlösung hergestellt, so dass der Neutralisationsgrad 75,0 mol-% betrug. Die Monomerlösung wurde nach dem Mischen der Komponenten durch einen Wärmetauscher kontinuierlich auf eine Temperatur von 30 °C abgekühlt und mit Stickstoff entgast. Als mehrfach ethylenisch ungesättigter Vernetzer wurde 3-fach ethoxyliertes Glycerylintriacrylat (ca. 85 gew.-%ig) verwendet. Die Einsatzmenge, bezogen auf die eingesetzte Acrylsäure (baAS), betrug 0,39 Gew.-%. Zur Initiierung der radikalischen Polymerisation wurden folgende Komponenten eingesetzt: 0,002 Gew.-% baAS Wasserstoffperoxid, zudosiert als 2,5 gew.-%ige wässrige Lösung, 0,1 Gew.-% baAS Natriumperoxodisulfat, zudosiert als 15 gew.-%ige wässrige Lösung, sowie 0,01 Gew.-% baAS Ascorbinsäure, zudosiert als 0,5 gew.-%ige wässrige Lösung. Der Durchsatz der Monomerlösung betrug 800 kg/h.

Die einzelnen Komponenten wurden kontinuierlich in einen Reaktor vom Typ List ORP 250 Contikneter (List AG, Arisdorf, Schweiz) eindosiert. Im ersten Drittel des Reaktors wurden zusätzlich 26,3 kg/h abgetrenntes Feinkorn mit einer Partikelgröße kleiner 150 $\mu$m zugesetzt, das bei der Klassierung nach Mahlung und Trocknung des Polymers erhalten wurde.

Die Reaktionslösung hatte am Zulauf eine Temperatur von 30 °C. Die Verweilzeit der Reaktionsmischung im Reaktor betrug ca. 15 Minuten.

Das so erhaltene Polymergel wurde mit einem Extruder vom Typ ECT EXR T80 (ECT GmbH, Mühlacker, Deutschland) extrudiert. Die Temperatur desPolymergels bei der Extrusion betrug 86 °C. Der Extrudermantel wurde mit 150 °C heißem Öl beheizt. Die Lochplatte hatte 66 Löcher mit einem Lochdurchmesser von 8 mm. Die Dicke der Lochplatte betrug 33 mm. Das Verhältnis von Innenlänge zu Innendurchmesser des Extruders (L/D) betrug 4. Die Rotationsgeschwindigkeit der Extruderschnecke betrug 100 Umdrehungen pro Minute.

Das Polymergel wurde auf Trockenbleche mit Siebboden (Maschenweite 250 $\mu$m, Drahtdurchmesser 130 $\mu$m) verteilt und 70 min bei 175 °C im Umlufttrockenschrank getrocknet. Die Beladung der Trockenbleche mit Polymergel betrug 0,81 g/cm².

Das getrocknete Polymergel wurde mittels eines einstufigen Walzenstuhles gemahlen (drei Mahlgänge, 1. Mahlgang mit Spaltbreite 2000 $\mu$m, 2. Mahlgang mit Spaltbreite 600 $\mu$m und 3. Mahlgang mit Spaltbreite 400 $\mu$m). Das gemahlene getrocknete Polymergel wurde klassiert und eine synthetische Partikelgrößenverteilung (PSD) mit folgender Zusammensetzung abgemischt:

| | |
|---|---|
| 500 bis 710 $\mu$m: | 5,9 Gew.-% |
| 300 bis 500 $\mu$m: | 57,8 Gew.-% |
| 150 bis 300 $\mu$m: | 36,3 Gew.-% |

Ein Teil des Feinkorns (Siebfraktion mit einer Partikelgröße <150 $\mu$m) wurde verwendet, um es bei der Polymerisation in den Reaktor mit einzudosieren.

1200 g des getrockneten Polymers wurden zur Oberflächennachvernetzung in einem Pflugschar®-Mischer mit Heizmantel vom Typ M5 (Gebr. Lödige Maschinenbau GmbH, Paderborn, Deutschland) bei 23 °C und einer Wellendrehzahl von 200 Umdrehungen pro Minute mittels einer Zweistoff-Sprühdüse mit folgender Lösung beschichtet (jeweils bezogen auf das Grundpolymer):

| | |
|---|---|
| 0,994 Gew.-% | Isopropanol |
| 0,14 Gew.-% | einer Lösung aus 50 Gew.-% 1,3-Propandiol und 50 Gew.-% N-(2-Hydroxyethyl)-2-oxazolidinon |
| 0,431 Gew.-% | entsalztes Wasser |
| 0,70 Gew.-% | 1,2-Propandiol |
| 2,273 Gew.-% | einer 22 gew.-%igen wässrigen Aluminiumlaktatlösung |
| 0,004 Gew.-% | Sorbitan-Monolaurat (Span 20, Croda International PLC) |

Nach dem Aufsprühen wurde das Produkt aus dem Mischer entnommen und in einen zweiten Mischer desselben Typs, der mit 238 °C heißer Heizflüssigkeit vorgeheizt worden war, überführt. Die Wellendrehzahl wurde auf 50 Umdrehungen pro Minute eingestellt und das Produkt auf eine Produkttemperatur von 185 °C gebracht. Um diese Temperatur zu halten, wurde die Temperatur der Heizflüssigkeit passend reduziert. Dem Reaktionsgemisch wurden beginnend mit der 25. Minute nach Polymereinfüllen alle 5 Minuten Proben von jeweils etwa 20 g entnommen. Insgesamt wurden 10 Proben entnommen. Die Produktproben wurden über ein Sieb mit einer Maschenweite von 710 $\mu$m abgesiebt, um Agglomerate zu entfernen. Es wurden die SFC-Werte aller Proben bestimmt. Der Vergleichbarkeit halber wurden die Proben ausgewählt, deren SFC-Wert am nächsten bei 100 $(cm^3 \cdot s)/10^7$ g lag. Ihre Eigenschaften sind in Tabelle 1 angegeben.

Tabelle 1: Eigenschaften der Polymerpartikel

| # | FSR$_{gesamt}$ [g/g·s] | FSR$_{150-300\,\mu m}$ [g/g·s] | $\Delta$FSR [g/g·s] | CRC [g/g] | SFC [(cm$^3$·s)/(10$^7$ g)] | Anteil 150-300 $\mu$m [%] |
|---|---|---|---|---|---|---|
| Vgl. 1 | 0,23 | 0,33 | 0,10 | 26,0 | 101 | 27,7 |
| Vgl. 2 | 0,15 | 0,26 | 0,11 | 29,2 | 48 | 8,3 |
| Vgl. 3 | 0,17 | 0,26 | 0,09 | 29,5 | 51 | 27,2 |
| | | | | | | |
| Bsp. 1 | 0,28 | 0,44 | 0,16 | 26,2 | 106 | 25,6 |
| Bsp. 2 | 0,27 | 0,45 | 0,18 | 27,6 | 104 | 36,3 |

Tabelle 1 zeigt, dass in den erfindungsgemäßen Beispielen ein höherer $\Delta$FSR-Wert vorliegt, bedingt durch eine relativ stark erhöhte Quellgeschwindigkeit FSR$_{150-300\,\mu m}$ der Polymerpartikel mit einer Partikelgröße von 150 bis 300 $\mu$m und eine leicht erhöhte Quellgeschwindigkeit FSR$_{gesamt}$ der Gesamtheit der Polymerpartikel. Die höhere Differenz $\Delta$FSR führt zu einer verbesserten Flüssigkeitsverteilung bei segregierten Partikelschüttungen, in denen sich größere Partikel oben und kleinere Partikel unten absetzen, wie zum Beispiel in Windeln. Die Polymerpartikel zeigen dabei eine ausreichend hohe Zentrifugenretentionskapazität CRC und eine verbesserte Permeabilität SFC.

**Patentansprüche**

**1.** Wasserabsorbierende Polymerpartikel, aufgebaut aus vernetzten Polymerketten, die überwiegend einpolymerisierte Einheiten von Acrylsäure umfassen, die zumindest teilweise neutralisiert ist, **gekennzeichnet durch**

a) einen Anteil von Partikeln mit einer Partikelgröße von 150 bis 300 $\mu$m von mindestens 20 Gew.-%,
b) eine Quellgeschwindigkeit der Gesamtheit der Polymerpartikel FSR$_{gesamt}$ von mindestens 0,25 g/g·s,

c) eine Differenz ΔFSR von mindestens 0,15, wobei ΔFSR definiert ist als

$$\Delta FSR = FSR_{150\text{-}300\,\mu m} - FSR_{\text{gesamt}} \quad ,$$

worin $FSR_{150\text{-}300\,\mu m}$ für die Quellgeschwindigkeit der Polymerpartikel mit einer Partikelgröße von 150 bis 300 μm steht,
d) eine Zentrifugenretentionskapazität CRC von mindestens 25,0 g/g, und
e) eine Permeabilität des gequollenen Gelbetts SFC von mindestens $90 \times 10^{-7}$ $CM^3$·s/g. wobei die Parameter mit den in der Beschreibung genannten Messmethoden bestimmmt wurden.

2. Polymerpartikel nach Anspruch 1, **gekennzeichnet durch** einen Anteil von Partikeln mit einer Partikelgröße von 150 bis 300 μm von mindestens 25 Gew.-%.

3. Polymerpartikel nach einem der vorhergehenden Ansprüche, **gekennzeichnet durch** einen Anteil von Partikeln mit einer Partikelgröße von 150 bis 300 μm von höchstens 80 Gew.-%.

4. Polymerpartikel nach einem der vorhergehenden Ansprüche, **gekennzeichnet durch** einen Anteil von Partikeln mit einer Partikelgröße von weniger als 150 μm von höchstens 5 Gew.-%.

5. Polymerpartikel nach einem der vorhergehenden Ansprüche, **gekennzeichnet durch** einen Anteil von Partikeln mit einer Partikelgröße von mehr als 710 μm von höchstens 5 Gew.-%.

6. Polymerpartikel nach einem der vorhergehenden Ansprüche, **gekennzeichnet durch** eine Quellgeschwindigkeit der Gesamtheit der Polymerpartikel $FSR_{\text{gesamt}}$ von mindestens 0,26 g/g·s.

7. Polymerpartikel nach einem der vorhergehenden Ansprüche, **gekennzeichnet durch** eine Quellgeschwindigkeit der Polymerpartikel mit einer Partikelgröße von 150 bis 300 μm $FSR_{150\text{-}300\,\mu m}$ von mindestens 0,35 g/g·s.

8. Polymerpartikel nach einem der vorhergehenden Ansprüche, **gekennzeichnet durch** eine Quellgeschwindigkeit der Polymerpartikel mit einer Partikelgröße von 150 bis 300 μm $FSR_{150\text{-}300\,\mu m}$ von mindestens 0,40 g/g·s.

9. Polymerpartikel nach einem der vorhergehenden Ansprüche, **gekennzeichnet durch** eine Zentrifugenretentionskapazität CRC von mindestens 26,0 g/g.

10. Polymerpartikel nach einem der vorhergehenden Ansprüche, **gekennzeichnet durch** eine Permeabilität des gequollenen Gelbetts SFC von mindestens $100 \times 10^{-7}$ $CM^3$·s/g.

11. Hygieneartikel, enthaltend wasserabsorbierende Polymerpartikel nach einem der vorhergehenden Ansprüche.

## Claims

1. Water-absorbing polymer particles formed from crosslinked polymer chains comprising predominantly polymerized units of at least partly neutralized acrylic acid, **characterized by**

a) a proportion of particles having a particle size of 150 to 300 μm of at least 20% by weight,
b) a free swell rate of the totality of the polymer particles $FSR_{\text{overall}}$ of at least 0.25 g/g·s,
c) a difference ΔFSR of at least 0.15, where ΔFSR is defined as

$$\Delta FSR = FSR_{150\text{-}300\,\mu m} - FSR_{\text{overall}}$$

in which $FSR_{150\text{-}300\,\mu m}$ represents the free swell rate of the polymer particles having a particle size of 150 to 300 μm,
d) a centrifuge retention capacity CRC of at least 25.0 g/g, and
e) a permeability of the swollen gel bed SFC of at least $90 \times 10^{-7}$ cm³·s/g,

where the parameters have been determined by the test methods cited in the description.

2.  Polymer particles according to claim 1, **characterized by** a proportion of particles having a particle size of 150 to 300 µm of at least 25% by weight.

3.  Polymer particles according to either of the preceding claims, **characterized by** a proportion of particles having a particle size of 150 to 300 µm of at most 80% by weight.

4.  Polymer particles according to any of the preceding claims, **characterized by** a proportion of particles having a particle size of less than 150 µm of at most 5% by weight.

5.  Polymer particles according to any of the preceding claims, **characterized by** a proportion of particles having a particle size of more than 710 µm of at most 5% by weight.

6.  Polymer particles according to any of the preceding claims, **characterized by** a free swell rate of the entirety of the polymer particles $FSR_{overall}$ of at least 0.26 g/g·s.

7.  Polymer particles according to any of the preceding claims, **characterized by** a free swell rate of the polymer particles having a particle size of 150 to 300 µm $FSR_{150-300\ \mu m}$ of at least 0.35 g/g·s.

8.  Polymer particles according to any of the preceding claims, **characterized by** a free swell rate of the polymer particles having a particle size of 150 to 300 µm $FSR_{150-300\ \mu m}$ of at least 0.40 g/g·s.

9.  Polymer particles according to any of the preceding claims, **characterized by** a centrifuge retention capacity CRC of at least 26.0 g/g.

10. Polymer particles according to any of the preceding claims, **characterized by** a permeability of the swollen gel bed SFC of at least $100 \times 10^{-7}$ cm$^3$·s/g.

11. A hygiene article comprising water-absorbing polymer particles according to any of the preceding claims.

**Revendications**

1.  Particules polymères absorbant l'eau, formées à partir de chaînes polymères réticulées, qui comprennent principalement des unités copolymérisées d'acide acrylique qui est au moins partiellement neutralisé, **caractérisées par** :

    a) une proportion de particules ayant une taille de particule de 150 à 300 µm d'au moins 20 % en poids,
    b) une vitesse de gonflement de la totalité des particules polymères $FSR_{total}$ d'au moins 0,25 g/g·s,
    c) une différence ΔFSR d'au moins 0,15, ΔFSR étant défini par :

    $$\Delta FSR = FSR_{150-300\ \mu m} - FSR_{total},$$

    $FSR_{150-300\ \mu m}$ représentant la vitesse de gonflement des particules polymères ayant une taille de particule de 150 à 300 µm,
    d) une capacité de rétention centrifuge CRC d'au moins 25,0 g/g et
    e) une perméabilité du lit de gel gonflé SFC d'au moins $90 \times 10^{-7}$ cm$^3$·s/g,

    les paramètres ayant été déterminés par les méthodes de mesure indiquées dans la description.

2.  Particules polymères selon la revendication 1, **caractérisées par** une proportion de particules ayant une taille de particule de 150 à 300 µm d'au moins 25 % en poids.

3.  Particules polymères selon l'une quelconque des revendications précédentes, **caractérisées par** une proportion de particules ayant une taille de particule de 150 à 300 µm d'au plus 80 % en poids.

4.  Particules polymères selon l'une quelconque des revendications précédentes, **caractérisées par** une proportion de particules ayant une taille de particule inférieure à 150 $\mu$m d'au plus 5 % en poids.

5. Particules polymères selon l'une quelconque des revendications précédentes, **caractérisées par** une proportion de particules ayant une taille de particule supérieure à 710 $\mu$m d'au plus 5 % en poids.

6. Particules polymères selon l'une quelconque des revendications précédentes, **caractérisées par** une vitesse de gonflement de la totalité des particules polymères $FSR_{total}$ d'au moins 0,26 g/g·s.

7. Particules polymères selon l'une quelconque des revendications précédentes, **caractérisées par** une vitesse de gonflement des particules polymères ayant une taille de particule de 150 à 300 $\mu$m $FSR_{150-300\ \mu m}$ d'au moins 0,35 g/g·s.

8. Particules polymères selon l'une quelconque des revendications précédentes, **caractérisées par** une vitesse de gonflement des particules polymères ayant une taille de particule de 150 à 300 $\mu$m $FSR_{150-300\ \mu m}$ d'au moins 0,40 g/g·s.

9. Particules polymères selon l'une quelconque des revendications précédentes, **caractérisées par** une capacité de rétention centrifuge CRC d'au moins 26,0 g/g.

10. Particules polymères selon l'une quelconque des revendications précédentes, **caractérisées par** une perméabilité du lit de gel gonflé SFC d'au moins 100 x $10^{-7}$ $cm^3$·s/g.

11. Article d'hygiène, contenant des particules polymères absorbant l'eau selon l'une quelconque des revendications précédentes.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 01038402 A1
- WO 03022896 A1
- WO 03051415 A1
- WO 2006034806 A1
- WO 2006034853 A1
- DE 102006037983 A
- BE 1020479 A
- WO 2005097313A A
- WO 2009115472 A1
- EP 0497623 A2
- WO 2005097313 A1
- EP 2014050980 W
- WO 02055469 A1
- WO 03078378 A1
- WO 2004035514 A1
- EP 0530438 A1
- EP 0547847 A1
- EP 0559476 A1
- EP 0632068 A1
- WO 9321237 A1
- WO 03104299 A1
- WO 03104300 A1
- WO 03104301 A1
- DE 10331450 A1
- DE 10331456 A1
- DE 10355401 A1
- DE 19543368 A1
- DE 19646484 A1
- WO 9015830 A1
- WO 02032962 A2
- CH 664704 A5
- EP 0517068 A1
- WO 9712666 A1
- DE 2123956 A1
- EP 0603525 A1
- DE 19536944 A1
- DE 4118884 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 03031482 A1
- DE 3713601 A1
- EP 0640330 A1

**In der Beschreibung aufgeführte Nicht-Patentliteratur**

- **F.L. BUCHHOLZ ; A.T. GRAHAM.** Modern Super-absorbent Polymer Technology. Wiley-VCH, 1998, 71-103